# EUROPEAN PATENT APPLICATION

(11) **EP 2 145 587 A2**
(43) Date of publication of application: **20.01.2010**
(21) Application number: 09251790.3
(22) Date of filing: 14.07.2009
(51) Int. Cl.: A61B 17/06

(54) **Differentiation of surgical filaments**

(30) Priority: 14.07.2008 US 80317 P; 08.07.2009 US 499300
(71) Applicant: Tyco Healthcare Group LP, North Haven, CT 06473 (US)
(72) Inventor: Abuzaina, Ferass, Shelton, CT 06484 (US); Hotter, Joseph, Middletown, CT 06457 (US)
(74) Representative: Soames, Candida Jane

(57) **Abstract**

The present disclosure relates to a filament for use in surgical procedures. The filament includes a first end with a first tangible indicator and a second end with a second, different tangible indicator. The first and second tangible indicators attribute different surface topographies to the respective first and second ends of the filament to provide a way of differentiating therebetween through tactile engagement.

## Description

### CROSS-REFERENCE TO RELATED APPLICATIONS

This application claims the benefit of U.S. Provisional Application No. 61/080,317, filed on July 14, 2008, the entire contents of which are incorporated herein by reference.

### BACKGROUND

### 1. Technical Field

The present disclosure relates generally to apparatus for securing together tissue, and methods of manufacturing the same. More particularly, the present disclosure relates to a surgical filament incorporating different tangible indicators at least one end to allow a clinician to differentiate therebetween during use.

### 2. Background of the Related Art

In surgical procedures, sutures are used to repair openings in skin, internal organs, blood vessels, and the like, as in the case of meniscal repair, and to join various tissues together, as in the reattachment of ligaments or tendons to bone. Generally, surgical procedures can be categorized as either open procedures or minimally invasive procedures.

Minimally invasive procedures, e.g., arthroscopic, endoscopic, and laparoscopic procedures, are generally performed through small openings in a patient's tissue, such as a natural opening in the body or an incision created through the use of a scalpel. The presence of tissue, blood, and other fluids at the surgical worksite can result in decreased visibility and a narrowed field of vision. As a result, it can be difficult for the clinician to locate a suture, ascertain its orientation, and/or distinguish between its ends should the suture become entangled during use. This difficulty is of course increased in those procedures requiring the simultaneous use of more than one suture.

Various advances have been made in an attempt to cope with decreased visibility at the surgical worksite. For example, some sutures include one or more colored sections that can help the clinician discern between different sutures and their ends. However, there are limitations regarding which colors can be feasibly distinguished from one another in the presence of fluids and tissue. Sutures have also been developed that include physical characteristics the clinician can perceive through the sense of touch, rather than sight. For example, U.S. Patent Application Publication No. 2007/0225763 to Zwolinski et al. (hereinafter "Zwolinski"), filed on March 23, 2006, describes the use of physical markings on the outer surface of a suture. While the markings suggested by Zwolinski may provide information regarding, the amount of suture material, the length of the suture, the depth of suture penetration, or the tension being applied to the suture, improvements in the field are desired to allow the clinician to distinguish one end of the suture from the other.

### SUMMARY

In one aspect of the present disclosure, a surgical filament is disclosed for joining tissue during a surgical procedure. The filament has first and second ends including tangible structure that facilitates differentiation therebetween through tactile engagement. The tangible structure includes a first tangible indicator at the first end of the filament and a second tangible indicator at the second end of the filament. The first and second tangible indicators define differing surface topographies, and as such, the filament defines a configuration that varies between the first and second ends thereof.

In one embodiment, the first and second tangible indicators include at least one protrusion extending outwardly of an outer surface of the filament. The at least one protrusion of the first tangible indicator may define a configuration that is different from that of the at least one protrusion of the second tangible indicator.

The first and second tangible indicators may include the same or differing numbers of protrusions. However, where the first and second tangible indicators include the same number of protrusions, the at least one protrusion of the first tangible indicator may define a dimension that is different from that of the at least one protrusion of the second tangible indicator.

The first and second tangible indicators may each include at least two protrusions, in which case the at least two protrusions of the first tangible indicator may define a distance therebetween that is different than the distance defined between the at least two protrusions of the second tangible indicator.

In an alternative embodiment, the first and second tangible indicators may each include at least one indentation extending into an outer surface of the filament. The at least one indentation of the first tangible indicator may define a configuration that is different from that of the at least one protrusion of the second tangible indicator.

The first and second tangible indicators may include the same or differing numbers of indentations. However, where the first and second tangible indicators include the same number of indentations, the at least one indentation of the first tangible indicator may define a dimension that is different from that of the at least one indentation of the second tangible indicator.

The first and second tangible indicators may each include at least two indentations, in which case, the at least two indentations of the first tangible indicator may define a distance therebetween that is different than the distance defined between the at least two indentations of the second tangible indicator.

The filament further includes an elongate body, or central portion, extending between the first and second ends. The elongate body may define a surface topography that differs from that defined by the tangible structure included on at least one of the first and second ends of the surgical filament.

In another embodiment, one of the first and second tangible indicators defines a surface topography that is substantially uniform.

The filament may further include an anchor member connected to either, or both of the first and second ends that is configured to limit movement towards a distal portion upon insertion into tissue.

In one embodiment of the filament, the tangible structure includes one or more projections on each of the first and second ends. The projections may be present at each end of the filament in the same or different numbers. To facilitate differentiation between the first and second ends through tactile engagement, the projections included on the first end may differ in number and/or configuration from the projections included on the second end. For example, the projections included on the first end may be configured as loops, whereas the projections included on the second end may be configured as arrow-shaped members. Where the first and second ends of the filament include differing numbers of projections, the projections included on the first and second ends may define the same configuration.

In another aspect of the present disclosure, a surgical suture for joining tissue during a surgical procedure is disclosed. The suture includes at least one surgical fiber and at least one surgical filament arranged so as to define a plurality of interstices therebetween. The at least one surgical filament has first and second ends including tangible structure that allows a clinician to differentiate between the ends of the suture through tactile engagement.

The present disclosure also relates to a method of manufacturing a surgical filament for joining tissue during a surgical procedure. The method includes the steps of providing a surgical filament having a first end and a second end, and forming tangible structure at each of the first and second ends to facilitate differentiation therebetween through tactile engagement.

The present disclosure further relates to a method of manufacturing a surgical suture for joining tissue during a surgical procedure. The method includes the steps of providing at least one surgical fiber, providing at least one surgical filament having a first end and a second end, forming tangible structure at each of the first and second ends of the filament, and arranging the at least one fiber and the at least one filament so as to define a plurality of interstices therebetween. Including the tangible structure on each of the first and second ends of the at least one filament allows a clinician to differentiate between ends of the suture through tactile engagement.

### BRIEF DESCRIPTION OF THE DRAWINGS

Various embodiments of the present disclosure are described herein below with reference to the drawings, wherein:

FIG. 1 is a side, plan view of one embodiment of a filament for use during a surgical procedure with first and second ends having first and second tangible indicators, respectively, that each include at least one protrusion;

FIG. 2 is a side plan view of another embodiment of a filament of the present disclosure;

FIG. 2A is an end view of the first end of the filament shown in FIG. 2 illustrating the first tangible indicator;

FIG. 2B is an end view of the second end of the filament shown in FIG. 2 illustrating the second tangible indicator;

FIG. 3 is a side, plan view of an alternative embodiment of a filament in accordance with the present disclosure;

FIG. 4 is a side, plan view of another embodiment of a filament in accordance with the present disclosure;

FIG. 4A is an end view of a first end of the filament shown in FIG. 4 illustrating a first tangible indicator;

FIG. 4B is an end view of a second end of the filament shown in FIG. 4 illustrating a second tangible indicator;

FIG. 5 is a side, plan view of another alternative embodiment of the filament in accordance with the present disclosure;

FIGS. 6-10 are side, plan views of the filament shown in of FIG. 1 illustrating alternative configurations for the protrusions of the first and second tangible indicators;

FIG. 11 is a side, plan view of another embodiment of a filament of the present disclosure with first and second ends having first and second tangible indicators, respectively, that each include at least one indentation;

FIG. 12 is a side, plan view illustrating the embodiment of the filament shown in FIG. 11;

FIG.12A is an end view of a first end of the filament shown in FIG. 12 illustrating the first tangible indicator;

FIG. 12B is an end view of a second end of the filament shown in FIG. 12 illustrating the second tangible indicator;

FIG. 13 is a side, plan view of an alternative embodiment of a filament in accordance with the present disclosure;

FIG. 14 is a side, plan view of still another embodiment of a filament in accordance with the present disclosure;

FIG.14A is an end view of a first end of the filament shown in FIG. 14 illustrating the first tangible indicator;

FIG. 14B is an end view of a second end of the filament shown in FIG. 14 illustrating the second tangible indicator;

FIG. 15 is a side, plan view of an alternative embodiment of a filament in accordance with the present disclosure;

FIGS. 16-20 are side, plan views of a filament illustrating alternative configurations for the indentations of the first and second tangible indicators;

FIGS. 21-24 are side, plan views of filaments in accordance with the present disclosure incorporating first and second tangible indicators that include projections associated with the ends of the filaments;

FIG. 25 is a side, plan view of an additional embodiment of a filament in accordance with the present disclosure including an elongate body, or central portion, that defines the same topography as one of the first and second ends of the filament;

FIG. 26 is a side, plan view of an alternative embodiment of a filament in accordance with the present disclosure;

FIG. 27 is a side, plan view of a suture for use during a surgical procedure that includes a plurality of fibers intertwined with a filament having first and second ends that include first and second tangible indicators, respectively;

FIG. 28 is a side, plan view of an alternative embodiment of the suture shown in FIG. 27;

FIG. 29 is a side, cross-sectional view of the filament shown in FIG. 1 shown in connection with a pair of anchor members; and

FIG. 30 is a perspective view of the filament shown in FIG. 1 in connection with a surgical needle and illustrating a method of attaching tissue segments.

### DETAILED DESCRIPTION

In the drawings and in the description which follows, in which like references characters identify similar or identical elements, the term "filament" should be understood as referring to any elongate member suitable for the intended purpose of joining tissue, including but not limited to sutures, ligatures, and surgical tape. In addition, the term "tissue" should be understood as referring to any bodily tissue, including but not limited to skin, fascia, ligaments, tendons, muscle, and bone.

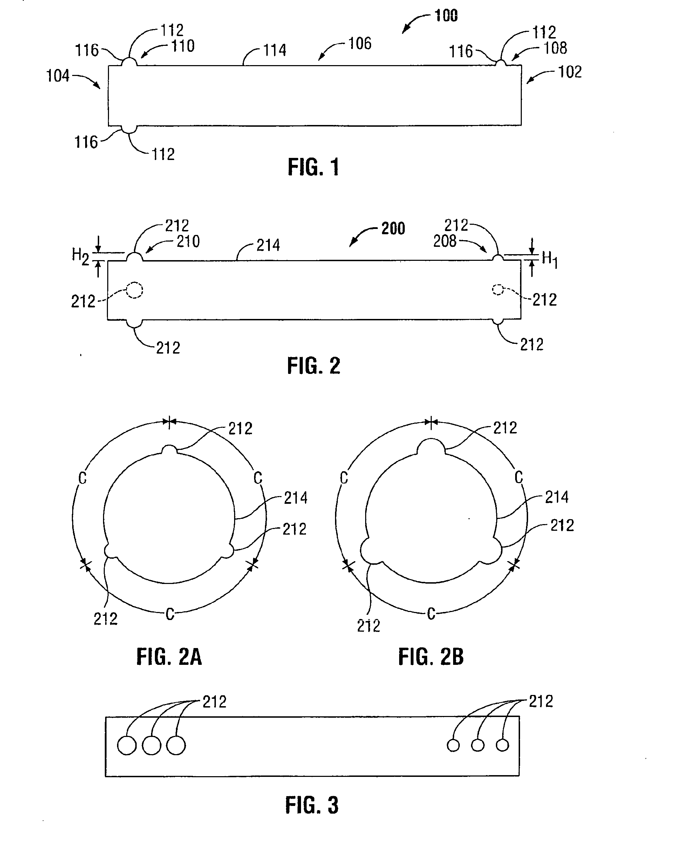

FIG. 1 illustrates one embodiment of a filament, referred to generally by character 100. The filament 100 can be formed from any suitable biodegradable or non-biodegradable material. Suitable biodegradable materials which may be used to construct a filament of the present disclosure include polymers such as those made from lactide, glycolide, caprolactone, valerolactone, carbonates (e.g., trimethylene carbonate, tetramethylene carbonate, and the like), dioxanones (e.g., 1,4-dioxanone), 1,dioxepanones (e.g., 1,4-dioxepan-2-one and 1,5-dioxepan-2-one), ethylene glycol, ethylene oxide, esteramides, γ-hydroxyvalerate, β-hydroxypropionate, alpha-hydroxy acid, hydroxybuterates, poly (ortho esters), hydroxy alkanoates, tyrosine carbonates, polyimide carbonates, polyimino carbonates such as poly (bisphenol A-iminocarbonate) and poly (hydroquinone-iminocarbonate), polyurethanes, polyanhydrides, polymer drugs (e.g., polydiflunisol, polyaspirin, and protein therapeutics) and copolymers and combinations thereof. Suitable natural biodegradable polymers include collagen, cellulose, poly (amino acids), polysaccharides, hyaluronic acid, silk gut, copolymers and combinations thereof. Suitable non-biodegradable materials which may be used to construct a filament of the present disclosure include fluorinated polymers (e.g., fluoroethylenes, propylenes, fluoroPEGs), polyolefins such as polyethylene, polyesters such as poly ethylene terepththalate (PET), nylons, polyamides, polyurethanes, silicones, ultra high molecular weight polyethylene (UHMWPE), polybutesters, polyaryletherketone, steel, copolymers and combinations thereof. Additionally, non-biodegradable polymers and monomers may be combined with each other and may also be combined with various biodegradable polymers and monomers to create a composite filament.

Desirably, the filament 100 has a measure of flexibility such that the filament 100 can be manipulated by a clinician to join adjacent sections of tissue together. As an illustrative example, the filament 100 may be used to repair or close an incision, wound, or the like using conventional suturing techniques.

The filament 100 includes a first end 102, a second end 104, and an elongate body, or central portion, 106 that extends therebetween. The first end 102 includes a first tangible indicator 108 and the second end 104 includes a second, different tangible indicator 110. The tangible indicators 108, 110 can include any structure that is tactually perceptible by a clinician. Incorporating different tangible indicators 108, 110 provides a way of differentiating between the ends 102, 104 of the filament 100 through tactile engagement.

In the embodiment of the filament 100 seen in FIG. 1, the respective first and second tangible indicators 108, 110 each include one or more raised protrusions 112 extending outwardly of an outer surface 114 of the filament 100. However, the tangible indicators 108, 110 differ in the number of protrusions 112 that they include. As shown, the first tangible indicator 108 includes a single protrusion 112, whereas the second tangible indicator 110 includes a pair of protrusions 112. It should be appreciated that the protrusions 112 may be present in either greater or fewer numbers in alternative embodiments of the filament 100.

FIGS. 2-24 illustrate various embodiments of the filament 100, each of which is similar to the filament 100 seen in FIG. 1, but for the specific topographies of their ends that are attributable to the incorporation of various tangible indicators. Accordingly, each embodiment of the filament 100 will only be discussed with respect to the tangible indicators that they include.

FIGS. 2-2B illustrate a filament 200 including first and second tangible indicators 208, 210, respectively, with one or more protrusions 212. In contrast to the filament 100 seen in FIG. 1, which includes indicators 108, 110 that have differing numbers of protrusions 112, the tangible indicators 208, 210 each include an identical number of protrusions 212. However, the tangible indicators 208, 210 are distinguished from one another through the inclusion of protrusions 212 that vary in size. Alternatively, the tangible indicators 208, 210 may include protrusions 212 that have different relative spacing or different configurations.

As seen in FIGS. 2-2B, in one embodiment, the tangible indicators 208, 210 each include three protrusions 212 that are spaced equally from each other by a distance "C". In this embodiment, the first indicator 208 and the second indicator 210 differ in the size of the protrusions 212 that they include. The first tangible indicator 208 includes protrusions 212 that extend outwardly from the outer surface 214 of the filament 200 to define a first height "H₁', whereas the second tangible indicator 210 includes three protrusions 212 that extend outwardly from the outer surface 214 to define a second, greater height "H₂". While illustrated as extending along the periphery of the filament 200, the protrusions 212 may alternatively extend along the length of the filament 200 such that they are spaced axially from one another, as seen in FIG. 3.

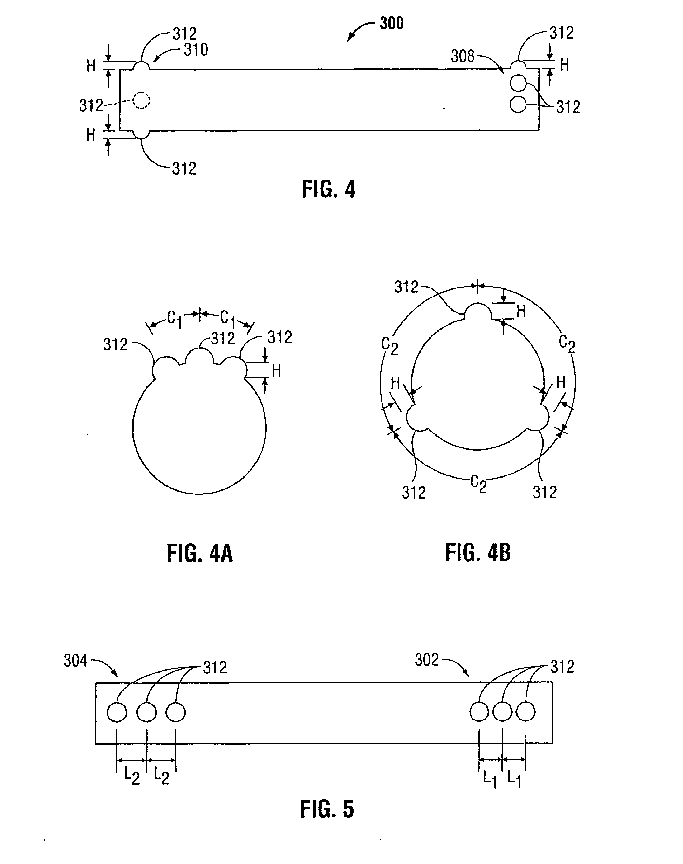

FIGS. 4-4B illustrate another embodiment of the filament, referred to as filament 300, with first and second tangible indicators 308, 310 that each include three protrusions 312 defining a substantially identical height "H". In this embodiment, the tangible indicators 308, 310 are distinguished by the distance or spacing between adjacent protrusions 312. The first tangible indicator 308 includes protrusions 312 that are spaced along the periphery of the filament 300 to define a first distance "C₁" therebetween, whereas the second tangible indicator 310 includes protrusions 312 that are spaced along the periphery of the filament 300 to define a second, greater distance "C₂" therebetween. Alternatively, as seen in FIG. 5, the protrusions 312 may be spaced linearly from each other to define a first distance "L₁" therebetween at the first end 302 of the filament 300 and a second, greater distance "L₂" therebetween at the second end 304.

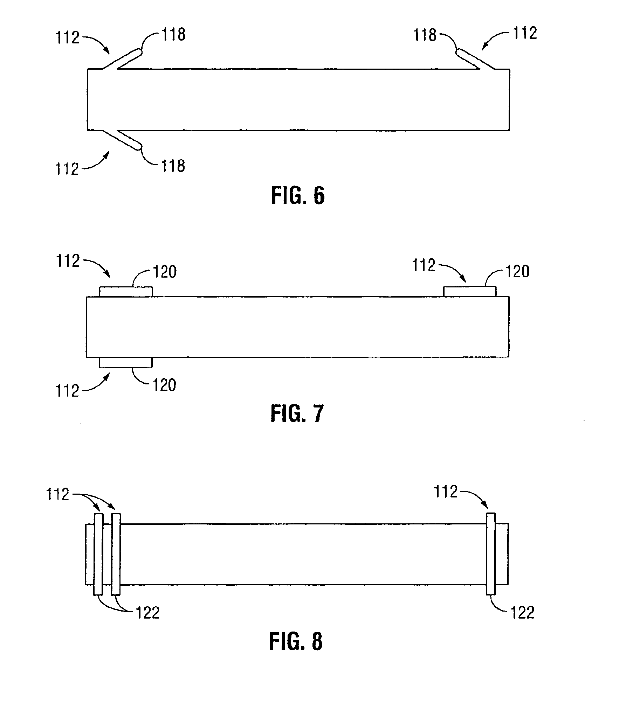

While the protrusions seen in FIGS. 1-5, e.g., protrusions 112 (FIG. 1), are depicted as substantially hemispherical protuberances 116, the protrusions 112 may define any configuration suitable for the intended purpose of providing structure that is tactually perceptible by a clinician. As illustrative examples, in alternative embodiments, the protrusions 112 may define fins 118, as seen in FIG. 6, linear ribs 120, as seen in FIG. 7, or annular ribs 122, as seen in FIG. 8.

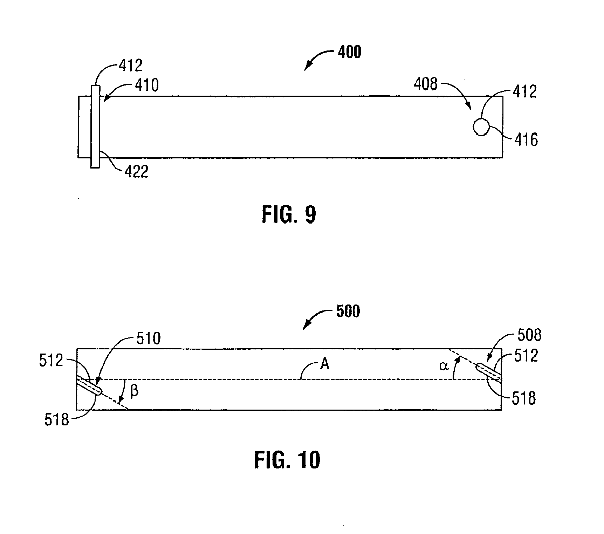

With reference now to FIG. 9, another embodiment of the filament, referred to as filament 400, is illustrated with first and second tangible indicators 408, 410, respectively, that include an equal number of protrusions 412. In this embodiment, the tangible indicators 408, 410 are distinguished by the configuration of the protrusions 412 that they include. For example, in the embodiment seen in FIG. 9, the first tangible indicator 408 includes a protrusion 412 configured as the aforedescribed protuberance 416, whereas the second tangible indicator 410 includes a protrusion 412 defining an annular rib 422. FIG. 10 illustrates another example of a filament 500 that includes a first tangible indicator 508 with a protrusion 512 defining a fin 518 that subtends a first angle α with respect to the longitudinal axis "A" defined by the filament 500, and a second tangible indicator 510 with a protrusion 512 defining a fin 518 that subtends a second, different angle β with respect to the longitudinal axis "A".

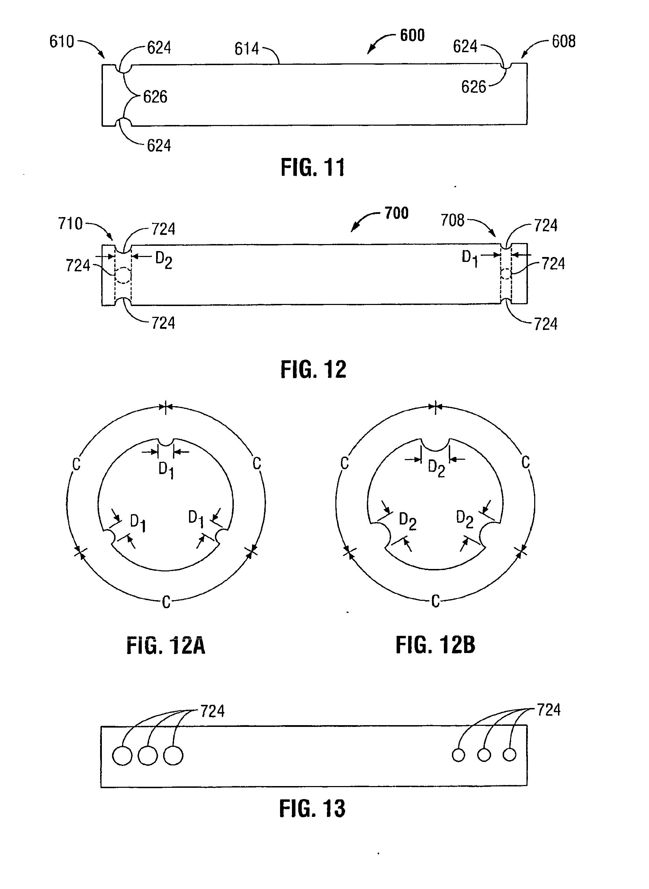

FIG. 11 illustrates yet another embodiment of the filament, referred to as filament 600, with first and second tangible indicators 608, 610, respectively, that each include one or more indentations 624 extending into the outer surface 614 of the filament 600. The first and second tangible indicators 608, 610 differ from each other, however, in the number of indentations 624 that they include. As shown, the first tangible indicator 608 includes a single indentation 624, whereas the second tangible indicator 610 includes a pair of indentations 624. It should be appreciated that the indentations 624 may be present in either greater or fewer numbers in alternative embodiments of the filament 600.

FIGS. 12-12B illustrate a filament 700 with first and second tangible indicators 708, 710 that also include one or more indentations 724. In contrast to the filament 600 seen in FIG. 11, which includes first and second tangible indicators 608, 610 having different numbers of indentations 612, the first and second tangible indicators 708, 710 of the filament 700 include an identical number of indentations 724 that vary in size. Alternatively, the tangible indicators 708, 710 may include indentations 724 that have different relative spacing or different configurations.

In one embodiment, the tangible indicators 708, 710 each include three indentations 724 spaced equally from each other by a distance "C". In this embodiment, the first indicator 708 and the second indicator 710 are distinguished from each other by the size of the indentations 724 that they include. The first tangible indicator 708 includes indentations 724 that define a first dimension "D₁", whereas the second tangible indicator 710 includes three indentations 724 that define a second, greater dimension "D₂". While illustrated as extending along the periphery of the filament 700, the indentations 724 may alternatively extend along the length of the filament 700 such that they are spaced axially from one another, as seen in FIG. 13.

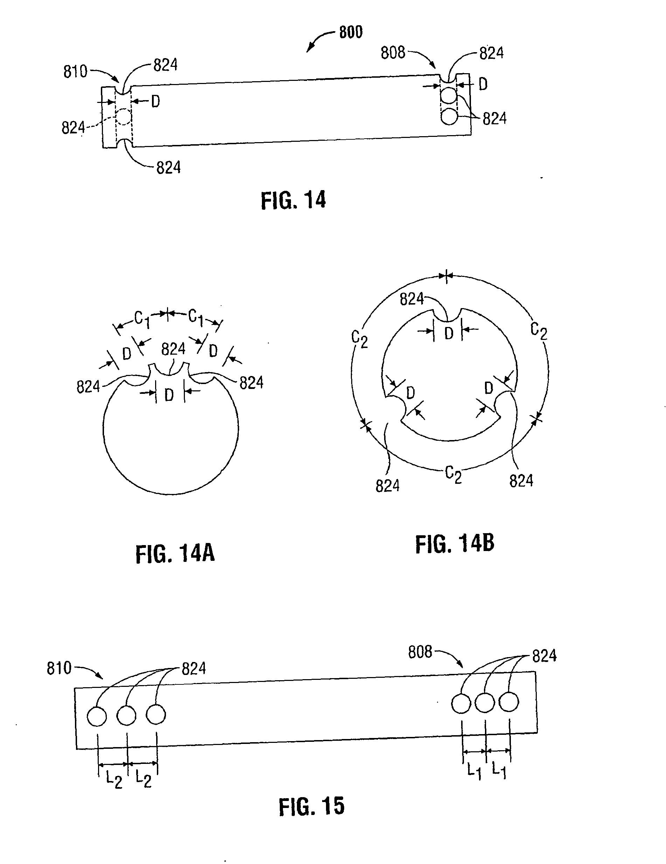

FIGS. 14-14B illustrate another embodiment, referred to as filament 800, with tangible indicators 808, 810 including indentations 824 that define a substantially identical dimension "D". In this embodiment, the tangible indicators 808, 810 are distinguished from each other by the distance between adjacent indentations 824. The first tangible indicator 808 includes indentations 824 that are spaced along the periphery of the filament 800 to define a first distance "C₁" therebetween, whereas the second tangible indicator 810 includes indentations 824 that are spaced along the periphery of the filament 800 to define a second, greater distance "C₂" therebetween. Alternatively, as seen in FIG. 15, the first tangible indicator 808 may include three indentations 824 that are spaced linearly from each other along the length of the filament 800 to define a first distance "L₁" therebetween, whereas the second tangible indicator 810 may include three protrusions 824 that are spaced from each other by a second, greater distance "L₂".

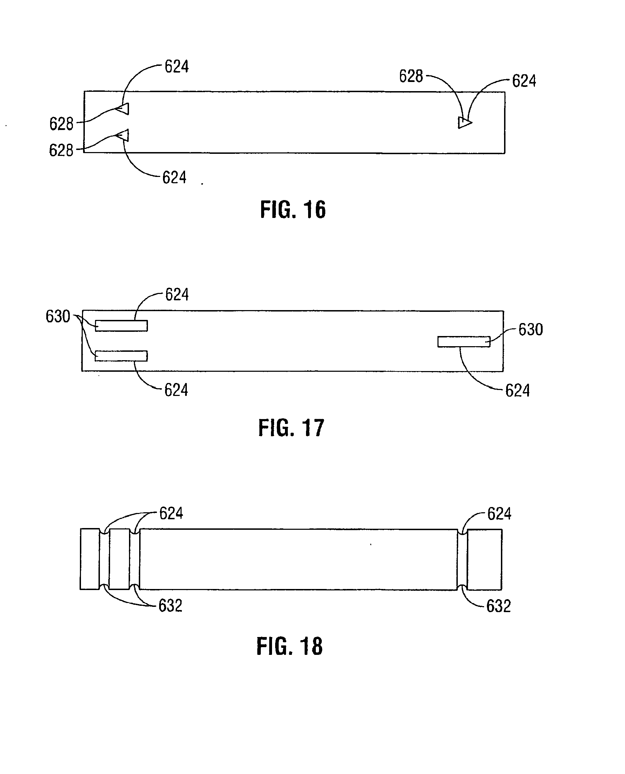

While the indentations seen in FIGS. 11-15, e.g., indentations 624 (FIG. 11), are depicted as substantially hemispherical depressions 626, the protrusions 624 may define any configuration suitable for the intended purpose providing structure that is tactually perceptible by a clinician. As illustrative examples, the indentations 624 may define substantially triangular configurations 628, as seen in FIG. 16, linear channels 630, as seen in FIG. 17, or arcuate channels 632, as seen in FIG. 18.

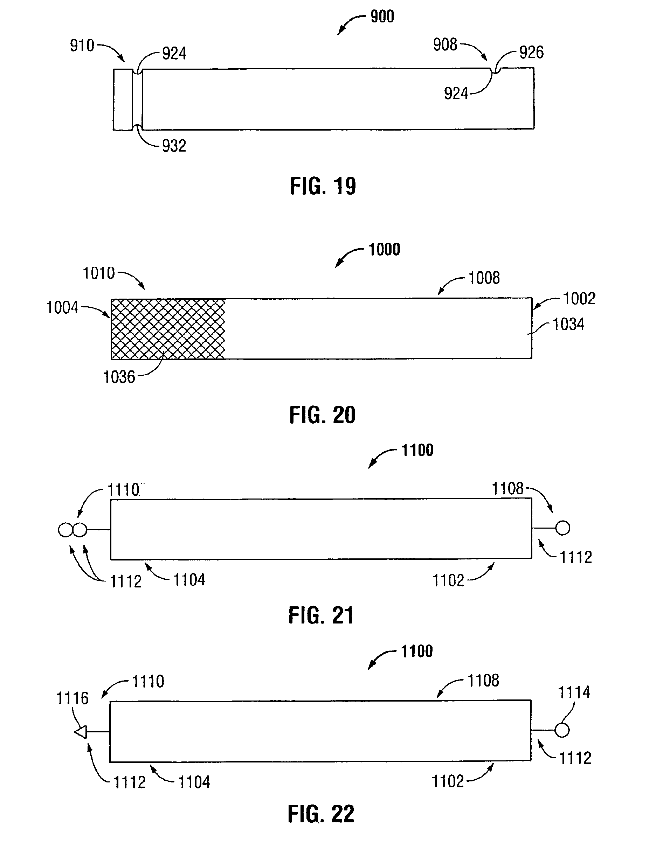

With reference now to FIG. 19, another embodiment of the filament, referred to as filament 900, is illustrated having a first tangible indicator 908 and a second tangible indicator 910 with an equal number of indentations 924. However, the tangible indicators 908, 910 differ in the configuration of the indentations 924 that they include. In the embodiment seen in FIG. 19, for example, the first tangible indicator 908 includes an indentation 924 defining the same substantially hemispherical depression 926 discussed above, whereas the second tangible indicator 910 includes an indentation 924 defining an arcuate channel 932.

With reference now to FIG. 20, an embodiment of the filament referred to by reference character 1000 is disclosed that includes first and second tangible indicators 1008, 1010, respectively. The first tangible indicator 1008 includes a substantially smooth surface 1034, whereas the second tangible indicator 1010 defines a knurled, or scarred surface 1036 such that a clinician may differentiate between the respective first and second ends 1002, 1004 of the filament 1000.

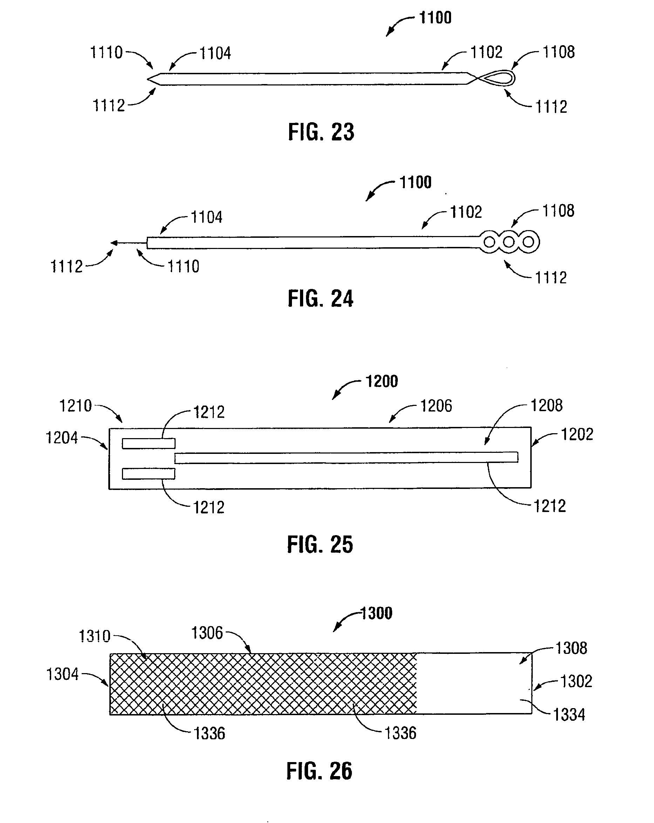

Turning now to FIGS. 21-24, additional embodiments of the filament will be discussed. The filament 1100 incorporates first and second tangible indicators 1108, 1110 that include projections 1112 extending beyond the first and second ends 1102, 1104 of the filament 1100, respectively. The projections 1112 may extend from the first and second ends 1102, 1104 of the filament 1100, as shown in FIGS. 21-22, for example. Alternatively, it is envisioned that either or both of the first and second tangible indicators 1108, 1110 may include projections 1112 that are integrally formed with the first and second ends 1102, 1104 of the filament 1100, respectively. For example, FIG. 23 illustrates the filament 1100 incorporating a first tangible indicator 1108 including a projection 1112 that extends from the first end 1102, and a second tangible indicator 1110 including a projection 1112 that is integrally formed with the second end 1104, whereas FIG. 24 illustrates the filament 1100 incorporating a first tangible indicator 1108 including a plurality of projections 1112 that are integrally formed with the first end 1102 and a second tangible indicator 1110 including a single projection 1112 that extends from the second end 1104.

To facilitate differentiation between the ends 1102, 1104 of the filament 1100 through tactile engagement, the first and second tangible indicators 1108, 1110 may include differing numbers of projections 1112, as shown in FIGS. 21 and 24. Referring specifically to the embodiment of the filament 1100 shown in FIG. 21 as an example, the first tangible indicator 1108 is illustrated as including a single projection 1112, whereas the second tangible indicator 1110 is illustrated as including a pair of projections 1112. Alternatively, the first and second tangible indicators 1108, 1110 may include the same number of projections 1112, as shown in FIGS. 22 and 23. In this case, to facilitate differentiation between the ends 1102, 1104 of the filament 1100, the first tangible indicator 1108 will include projections 1112 defining one configuration, and the second tangible indicator 1110 will include projections 1112 defining another, different configuration. Referring specifically to the embodiment of the filament 1100 shown in FIG. 22, the first tangible indicator 1108 includes a projection 1112 that is configured as a loop 1114, whereas the second tangible indicator 1110 includes a projection 1112 that is configured as an arrow-shaped member 1116. In alternative embodiments of the filament 1110, the projections 1112 may be configured in any manner suitable for the intended purpose of allowing a clinician to differentiate between the ends 1102, 1104 of the filament through tactile engagement.

While each of the aforedescribed embodiments has been illustrated as including an elongate body that exhibits a different topography than each of the first and second ends of the filament, e.g., the elongate body 106 of the filament 100 seen in FIG. 1 defines a substantially uniform topography in contrast to the textured topographies of the respective first and second ends 102, 104 attributable to the respective first and second tangible indicators 108, 110, a filament including a elongate body that exhibits the same topography as either of the first and second ends is also within the scope of the present disclosure. For example, FIG. 25 illustrates a filament 1200 that includes a first end 1202 with a first tangible indicator 1208 having a single, linear protrusion 1212, a elongate body 1206 that also includes the single, linear protrusion 1212, and a second end 1204 with a second tangible indicator 1210 having a pair of linear protrusions 1212. Alternatively, as seen in FIG. 26, a filament 1300 is disclosed that includes a first end 1302 having a first tangible indicator 1308 defining a substantially smooth surface 1334, as discussed above with respect to FIG. 20, an elongate body 1306 that includes a knurled surface 1336, and a second end 1304 having a second tangible indicator 1310 that also includes a knurled surface 1336.

Referring again to FIG. 1, a method of manufacturing the filament 100 will be described. Initially, a length of the filament 100 is provided, which may be varied dependent upon the intended use thereof. The tangible indicators 108, 110 are thereafter formed at the respective first and second ends 102, 104 of the filament 100. The tangible indicators 108, 110 may be formed through any suitable method, including but not being limited to molding, casting, extrusion, laser shaping, or mechanical processes, such as milling and/or grinding.

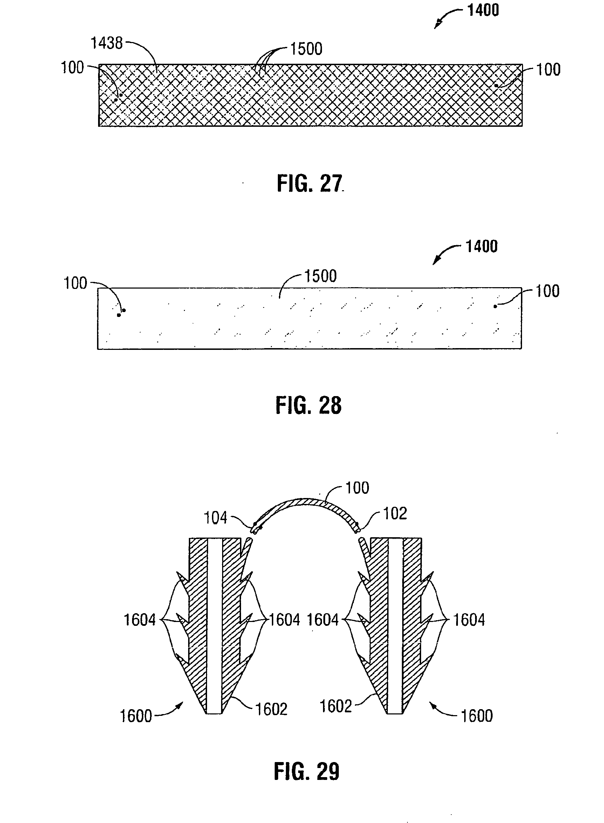

With reference now to FIGS. 27-28, a suture 1400 is disclosed. The suture 1400 includes one or more fibers 1500 commingled with any of the filaments discussed herein above. The filaments may be any of the filaments illustrated in the embodiments of FIGS. 1-25 or combinations thereof. For example, in one embodiment, the suture 1400 includes fibers 1500 and the filament 100 discussed with respect to FIG. 1. The fibers 1500 and the filament 100 are arranged to define a plurality of interstices 1438 therebetween, and may be arranged in any manner suitable for this intended purpose, including but not limited to braiding, entangling, weaving, or comingling of the fibers 1500 and the filament 100. The fibers 1500 and the filament 100 may be arranged in a braided configuration, as seen in FIG. 27, or alternatively, the fibers 1500 and the filament 100 may be loosely interwoven, as seen in FIG. 28. While the suture 1400 is illustrated as including only a single filament 100 among the fibers 1500, in alternative embodiments, the suture 1400 may include a greater number of filaments 100. As stated above, it is envisioned that suture 1400 may include any one or more of the filaments discussed above and is not limited to the embodiment shown.

To manufacture the suture 1400, initially, a length of the filament 100 (FIG. 1) is processed to define tangible indicators 108, 110 at the ends 102, 104 thereof, as previously described. Thereafter, the filament 100 and the fibers 1500 are arranged such that the plurality of interstices 1438 are defined therebetween.

Referring now to FIG. 29, any of the filaments discussed herein above, such as, for example, the filament 100 described with respect to FIG. 1, or the suture 1400 described with respect to FIGS. 27-28, may be used in conjunction with one or more anchor members 1600 of either the cannulated variety, as shown, or the non-cannulated variety. It is envisioned that any of the filaments described in any of the previous embodiments may be used in conjunction with the anchor members 1600 of the present embodiment such that the tangible indicators on the ends of the filament are exposed when the anchor member is buried into bone and/or tissue. The anchor members 1600 define a configuration that is at least partially tapered near their bottom ends 1602 to facilitate insertion thereof, although a non-tapered configuration is also within the scope of the present disclosure. In alternative embodiments, the bottom ends 1602 may be substantially blunt, as shown, or incisive to facilitate the penetration of tissue. In those embodiments, including multiple anchor members 1600, i.e., one anchor member 1600 that is attached to each end 102, 104 of the filament 100, the anchor members 1600 may be either substantially identical, as shown, or alternatively, the anchor members 1600 may define different dimensions and/or include different materials. Each anchor member 1600 includes at least one barb 1604 that is oriented so as to facilitate insertion of the anchor member 1600 into tissue and inhibit subsequent reversal thereof. Further details regarding the structure and/or function of the anchor members 1600 may be obtained through reference to commonly owned U.S. Patent No. 6,554,852 to Oberlander, the entire contents of which are incorporated by reference herein.

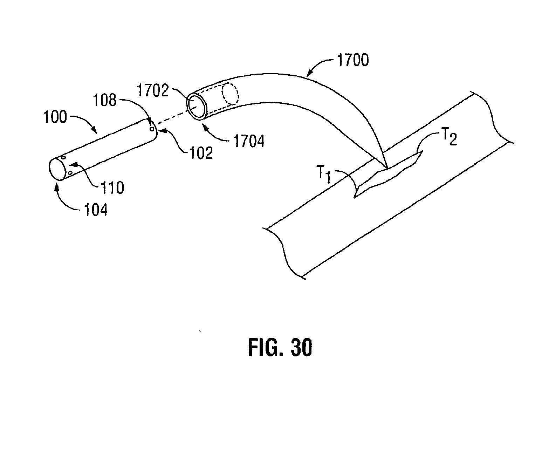

Referring now to FIG. 30, a method of using a surgical filament, e.g., the surgical filament 100 discussed above with respect to FIG. 1, to join adjacent tissue segments "T₁" and "T₂" will be discussed. To facilitate penetration of the tissue segments "T₁", "T₂", the surgical filament 100 is attached to a surgical needle 1700. The surgical filament 100 may be attached to the surgical needle 1700 through any suitable method, such as by inserting the first end 102 of the surgical filament into a bore 1702 provided at a proximal end 1704 of the surgical needle 1700, and thereafter crimping the proximal end 1704 of the surgical needle 1700. Alternative methods of attaching the surgical filament 100 and the surgical needle 1700, such as by integrally forming the surgical needle 1700 with the surgical filament 100, are also envisioned. The tissue segments "T₁", "T₂" may then be joined by passing the surgical needle 1700, and thus the surgical filament 100, through the tissue segments "T₁", "T₂" in any suitable manner. When visibility is limited, such as during the course of a procedure performed at an internal worksite for example, the different surface topographies of the tangible indicators 108, 110 will allow the practitioner to distinguish between the respective ends 102, 104 of the surgical filament 100. After the tissue segments "T₁", "T₂" have been successfully joined, the surgical needle 1700 can be removed from the surgical filament 100, and the surgical filament 100 can be tied.

Persons skilled in the art will understand that the devices and methods specifically described herein and illustrated in the accompanying drawings are intended to be construed as non-limiting, exemplary embodiments, and that the features illustrated or described in connection with one exemplary embodiment may be combined with the features of other embodiments. Additionally, one skilled in the art will appreciate further features and advantages of the disclosure based on the above-described embodiments. Accordingly, the disclosure is not to be limited by what has been particularly shown and described, except as indicated by the appended claims.

## Claims

1. A surgical filament for joining tissue during a surgical procedure, comprising:
first and second ends; and
a tangible structure included on at least one of the first and second ends to facilitate differentiation between the first and second ends through tactile engagement.

2. The filament recited in claim 1, wherein the tangible structure includes a first tangible indicator included on the first end of the filament and a second tangible indicator included on the second end of the filament, wherein the first and second tangible indicators each define differing surface topographies such that the filament defines a variable configuration between the first and second ends.

3. The filament recited in claim 2, wherein the first and second tangible indicators each include at least one protrusion extending outwardly of an outer surface of the filament.

4. The filament recited in claim 3, wherein the at least one protrusion of the first and second tangible indictors include at least one barb; or the filament recited in claim 3, wherein the at least one protrusion of the first tangible indicator defines a first configuration and the at least one protrusion of the second tangible indicator defines a second configuration, wherein the first configuration and the second configuration are different; or the filament recited in claim 3, wherein the first and second tangible indicators include differing numbers of protrusions; or the filament recited in claim 3, wherein the first and second tangible indicators include the same number of protrusions, wherein the at least one protrusion of the first tangible indicator defines a first dimension and the at least one protrusion of the second tangible indicator defines a second dimension, wherein the first dimension and the second dimension are different; or the filament recited in claim 3, wherein the first and second tangible indicators each include at least two protrusions, wherein the at least two protrusions of the first tangible indicator define a first distance therebetween and the at least two protrusions of the second tangible indicator define a second distance therebetween, wherein the first distance and the distance are different.

5. The filament recited in claim 2, wherein the first and second tangible indicators each include at least one indentation extending into an outer surface of the filament.

6. The filament recited in claim 5, wherein the at least one indentation of the first tangible indicator defines a first configuration and the at least one indentation of the second tangible indicator defines a second configuration, wherein the first configuration and the second configuration are different; or the filament recited in claim 5, wherein the first and second tangible indicators include differing numbers of indentations; or the filament recited in claim 5, wherein the first and second tangible indicators include the same number of indentations, wherein the first tangible indicator includes indentations defining a first dimension and the second tangible indicator includes indentations defining a second dimension, wherein the first dimension and the second dimension are different; or the filament recited in claim 5, wherein the first and second tangible indicators each include at least two indentations, wherein the at least two indentations of the first tangible indicator define a first distance therebetween and the at least two indentations of the second indicator define a second distance therebetween, wherein the first distance and the distance are different.

7. The filament recited in claim 1, further including an elongate body extending between the first and second ends, the elongate body defining a surface topography that differs from the surface topography defined by the tangible structure included on at least one of the first and second ends of the filament.

8. The filament recited in claim 2, wherein one of the first and second tangible indicators defines a surface topography that is substantially uniform.

9. The filament recited in claim 1, wherein the tangible structure included on each of the first and second ends of the filament includes at least one projection.

10. The filament recited in claim 9, wherein the tangible structure included on each of the first and second ends includes the same number of projections, wherein the at least one projection included on the first end of the filament differs in configuration from the at least one projection included on the second end of the filament.

11. The filament recited in claim 10, wherein the at least one projection included on the first end of the filament is configured as a loop and the at least one projection included on the second end of the filament is configured as an arrow-shaped member.

12. The filament recited in claim 9, wherein the number of projections included on the first end of the filament differs from the number of projections included on the second end of the filament.

13. The filament recited in claim 1, further including at least one needle attached thereto.

14. A multifilament suture comprising:
at least one filament including a first tangible indicator on a first end of the filament and a second tangible indicator on a second end of the filament, wherein the first and second tangible indicators each define differing surface topographies; and
at least one fiber commingled with the at least one filament.
